# EUROPEAN PATENT APPLICATION

(11) **EP 3 832 302 A1**
(43) Date of publication of application: **09.06.2021**
(21) Application number: 19213028.4
(22) Date of filing: 02.12.2019
(51) Int. Cl.: G01N 31/12, B01D 46/00, B01D 46/42, G01N 1/22, G01N 1/40

(54) **A HEATING CHAMBER FOR MEASURING CARBONACEOUS AEROSOL, AND A DEVICE COMPRISING SAID CHAMBER**

(71) Applicant: Aerosol d.o.o., 1000 Ljubljana (SI)
(72) Inventor: Rigler, Martin, 1293 Smarje - Sap (SI); Pilko, Viktor, 4000 Kranj (SI); Drinovec, Luka, 1000 Ljubljana (SI); Mocnik, Grisa, 1000 Ljubljana (SI)
(74) Representative: Patentni Biro AF d.o.o.

(57) **Abstract**

The present invention belongs to the field of systems adapted for detection and quantification of carbonaceous aerosol. It relates to an improved heating chamber for a device for measuring carbonaceous aerosol, the chamber comprising at least:
- an upper part and a lower part with a ring for closing and holes for pins through which heaters receive the voltage needed for heating;
- an inlet for leading the sampled air to a filter and a system of valves, which regulates the sampled air flow and air flow during the analysis;
- two heaters encasing the filter, each heater comprising at least a housing and a heating wire, wherein the first heater is installed in the upper part and the second heater is installed in the lower part, wherein the distance of the heaters from the filter is from 1 to 10 mm, and wherein the heaters are controlled with electronics; and
- an outlet for leading the created CO₂ towards a CO₂ detector.

## Description

### Field of the invention

The present invention belongs to the field of methods and devices for analysing materials by determining their chemical or physical properties by the use of thermosoptical means, more precisely to the systems especially adapted for detection and quantification of carbonaceous aerosol. The invention relates to an improved heating chamber for a device for measuring carbonaceous aerosol and a device comprising the said chamber.

### Background of the invention and the technical problem

Carbonaceous aerosols are extremely diverse and are frequently the largest and most important fraction of fine particulate matter mass (PM2.5) (Turpin et al., 2001, doi:10.1080/02786820119445; Solomon et al., 2008, J. Air & Waste Mgmt. Assn., 58, S3-S92). They impact air quality, visibility, climate forcing, cloud nucleation, the planetary radiation balance, and public health. The carbonaceous fractions are frequently separated into organic carbon (OC) and elemental carbon (EC) based on their volatility using thermal-optical methods. Although the combined measurement of total carbon (TC) concentration is usually reliable, (Karanasiou et al., 2015, doi:10.5194/amtd-8-9649-2015), the results for the separation of OC and especially EC fractions vary significantly for different thermal analysis methods (Schmid et al., 2001, Atmos. Environ., 35, 2111-2121,; tenBrink et al., 2004, doi.org/10.1016/j.atmosenv.2004.08.027; Bae et al., 2009, doi:10.1016/j.scitotenv.2009.05.035). 'Organic' (OC) compounds usually comprise the largest carbon-containing fraction of ambient aerosols: often more than 50% of the PM2.5 mass. A smaller fraction is categorized as Light-Absorbing Carbon ("LAC"), often described in terms of Black ("BC") and Brown ("BrC") Carbon (Petzold, 2013); or 'Elemental' Carbon ('EC'), which is defined instrumentally by thermal analysis methods.

Accurate, continuous and high time resolved data relating to TC are needed in order to assess the severity of the problem and to identify and investigate the main sources which require attention; and to quantitate the improvements following the application of controls and regulations. Devices for measuring carbonaceous aerosol, also called Total carbon analysers (TCA), have thus been developed, however their heaters used for combustion of sampled air heat the sample slowly. Therefore, it is the aim of the present invention to provide an improved chamber with heaters for a total carbon analyser device that will quickly reach temperatures around 1000 °C needed for reliable, repeatable and complete combustion. It is further desired that the chamber is robust and suitable for use in field measurements and experiments.

### State of the art

Conventional thermal analysis for the 'EC/OC' content of aerosols gives data that is highly dependent on the thermal analysis protocol that is used: 'NIOSH' vs. 'IMPROVE' vs. 'EUSAAR'. Commercially available OC/EC analysers have a quartz glass chamber, which is inert and resistant to high temperatures around 1000 °C. Heating of the sample is slow, as a hot catalyst MnO₂ is used for transformation of organic vapours into carbon dioxide, the quantity of which is then measured with suitable detectors, usually NDIR. One such analyser is described in patent application CN101963606 that discloses a combustion furnace for a total organic carbon automatic analyser, which comprises a glass component, a heating coil, a temperature sensor, a sample outlet, a gas outlet, a protective tube, a catalyst, a combustion furnace shell and heat-preservation cotton. The catalyst is arranged at the bottom of the glass component; the heating coil heats the sample to 900 °C under the catalysis of the catalyst; organic matters in the sample are combusted to generate carbon dioxide.

Solutions used in devices sold by companies Skalar, Eltra and Uic Inc. use different heater, where the heating wire is installed at a different location if compared to the present invention. Moreover, combustion is enabled by presence of a catalyst similarly as in commercially available OC/EC analysers. Hence, heating and consequently combustion of the analysed sample is not as efficient as in the present invention.

### Description of the solution of the technical problem

The essence of the improved chamber for an instrument for measuring carbonaceous aerosol is in that the airtight chamber comprises two heaters encasing a filter, wherein the chamber and the heaters are made of stainless steel, wherein the heaters are controlled by suitable electronics. Stainless steel is inert as the commercially available quartz chambers, but does not release any carbon at high temperatures, which could affect the measurements. Further, stainless steel is more robust than the fragile quartz glass, thus making the chamber resistant to impacts and suitable for field measurements and experiments. Both heaters comprise a housing and a heating wire, both made of material that does not release carbon upon heating, the preferred option being stainless steel. The material from which the heating wire is made should enable fast heating and should endure frequent heating and cooling. A preferred choice for the heating wire is material APM produced and sold by the company Kanthal. APM is an advanced powder-metallurgical, dispersion-strengthened, ferritic iron-chromium-aluminium alloy (FeCrAI alloy) for use at temperatures up to 1425°C. The alloy is characterized by excellent stability and oxidation resistance, has low tendency to ageing and low resistance change. Any material similar to the APM is suitable to be used for the heating wire of the heaters in the chamber according to the invention. The filter used in the chamber is preferably a quartz fibre filter. Suitable filters are all filter stable at temperatures up to 1100 °C. It would also be possible to use steel mesh filters with suitably small pores, however their collection efficiency is lower than with quartz filters. One of the said heaters is placed above the filter, while the other heater is placed below the filter, wherein the distance of the heaters from the filter is from 1 to 10 mm, preferably 1 to 5 mm. Such placement of heaters allows uniform and fast heating of the filter with the sample, which leads to efficient combustion of organic vapour into CO₂. The filter reaches its final temperature around 940 °C in approximately 10 seconds. The chamber is preferably equipped with a temperature sensor for measuring temperature below the filter, wherein the said sensor is usually placed in the lower part of the chamber. When the organic vapours evaporate from the filter, they reach the heating wires with temperatures around 900 °C almost immediately, meaning that they do not stick to the walls of the chamber before combustion. In case the evaporated vapours stick to the walls, they condense and become unavailable for detection with a suitable CO₂ sensor such as an NDIR sensor.

The stainless-steel chamber comprises at least the following:
- an upper part and a lower part with a ring for closing and holes for pins through which the heaters receive the voltage needed for heating, wherein all holes are provided with airtight seals, which withstand heating up to 300 C;
- a filter located between the upper and lower part;
- an inlet for leading the sampled air to the filter and a system of valves, which regulates the sampled air flow and air flow during the analysis;
- two heaters, each comprising at least a housing, two pins for connection with electronics controlling heating welded to a heating wire, wherein the first heater is installed in the upper part and the second heater is installed in the lower part, wherein the distance of the heaters from the filter is from X to W mm, and wherein the heaters are controlled with electronics comprising at least a voltage driver and voltage clamps; and
- an outlet for leading the created CO₂ out of the chamber towards a CO₂ detector.
The CO₂ detector located downstream of the chamber to measure the amount of CO₂ formed during combustion of sampled air enabled by the heaters, wherein the created CO₂ leaves the chamber through the outlet and reaches the detector via a solenoid or any other suitable connection.

The preferred embodiment of each heater comprises the steel housing also serving as the holder of the filter, wherein the following components are housed within the housing:
- the heating wire shaped as winding line to cover as much surface of the filter as possible;
- suitably shaped ceramics with small pins for protecting the heating wire from short circuits due to the metal (steel) housing; and
- at least one pin welded to the heating wire for connection to the electronics in order to allow heating of the heating pin by applying suitable voltage.
Said heaters are controlled by electronics, the main component of the latter being a voltage driver, one for each of the heaters. The voltage driver through suitable contact and ceramic pins regulates the voltage on the heating wire with 0.1 V precision, wherein the power of the heaters is measured with a voltage clamp. Said electronics are used to control operation of the heaters, wherein the lower heater is preferably turned on before the upper heater. As the sampled air travels downwards, the lower heater is turned on, thereby allowing the organic vapour to pass through an already hot heater, consequently turning them to CO₂. This allows efficient combustion and also enables abandonment of catalysts in the chamber. In case the upper heater would be turned on first, a portion of the organic vapour could escape through the lower heater having a temperature lower than required; hence this portion of vapour could not be detected with the CO₂ sensor. The heaters and consequently the filter heat to temperatures up to 1000 °C, wherein the chamber itself heats to temperatures up to 300 °C.

The chamber according to the invention is suitable for use in a device for quantification of carbonaceous aerosols, such as a Total Carbon Analyzer (TCA) instrument that uses a thermal method for total carbon (TC) determination. The TCA comprises at least one, preferably two parallel flow channels with two sampling-analytical heating chambers according to the invention, which alternate between sample collection and thermal analysis. While one channel is collecting its sample for the next time-base period, the other channel is analysing the sample collected during the previous period. This sequential feature offers the great advantage of a continuous measurement of TC. The sampling time may be pre-set from 20 minutes to 24 hours. At the end of the collection period, the sample flow is switched from one channel to the other.

The method of operation of the device with the chamber according to the invention comprises the following steps:
a) collecting a sample of atmospheric aerosols on the quartz fibre filter enclosed in the stainless-steel chamber, preferably at a controlled sampling flow rate of 16.7 LPM, wherein the sampling time is from 20 minutes to 24 hours, preferably 60 minutes,
b) combusting the sample from step a) with two flash-heating elements to convert all carbonaceous compounds into CO₂, wherein the first heater below the filter is turned on first and the second heater above the filter is turned on after the first heater, followed by a preferred step of adjusting the voltage on the first heater;
c) detecting in step b) created CO₂ by the NDIR CO₂ detector, wherein the background level of CO₂ in ambient air during the heating cycle is determined before and after the heating cycle to provide the baselines against which the combustion pulse is measured, and
d) cooling the chamber and combustion elements after analysis, wherein cooling is enabled with at least one fan located outside of the chamber in the device, where the chamber is installed.

A preferred embodiment of the step b) in the method of operation of the device with the chamber according to the invention is as follows:
i. heating of the first heater below the filter with a high voltage to achieve fast heating;
ii. heating of the second heater above the filter;
iii. adjusting the voltage on the first heater to achieve a temperature around 940 °C, which prevents overheating and unwanted degradation of the heating wire;
iv. turning of the second heater above the filter;
v. turning of the first heater below the filter; and
vi. cooling both heaters to a temperature below 50 °C.
Step iii) may also be performed immediately after step i) or at the same time as step ii).
Even more preferred embodiment repeats the above sequence of steps i) to vi). Such heating has an advantage that artefacts such as increased concentration of CO₂ detected by the CO₂ detector due to extremely fast heating of air are eliminated. The amount of CO₂ attributed to the carbonaceous aerosols collected on the filter is thus calculated as: CO₂ (Carbonaceous Aerosols) = CO₂ (first heating cycle) - CO₂ (second heating cycle).

The device with the chamber according to the invention can be used in online mode for continuous, real-time analysis and offline mode to analyse previously-collected samples. In case the device is linked to another device for measurement of black carbon (BC) such as Aethalometer AE33 (Aerosol, Slovenia), OC can be calculated as OC = TC (measured by the device) - BC (i.e. EC measured by AE33).

The invention will be further described based on embodiments and figures, which show:
- Figure 1: The upper part of the chamber according to the invention
- Figure 2: The bottom part of the chamber according to the invention
- Figure 3: An explosion view of the preferred embodiment of the heater
- Figure 4a: Operation of the device for measuring carbonaceous aerosol wherein the first chamber is performing analysis and the second chamber is sampling
- Figure 4b: Operation of the device for measuring carbonaceous aerosol wherein the first chamber is sampling and the second chamber is performing analysis
- Figure 5: Activity of both heaters, the temperature and CO₂ amount depending on the cycle stage
- Figure 6: Temperature of the filter in a regular heating cycle in the chamber
- Figure 7: The chamber according to the invention with the temperature sensor

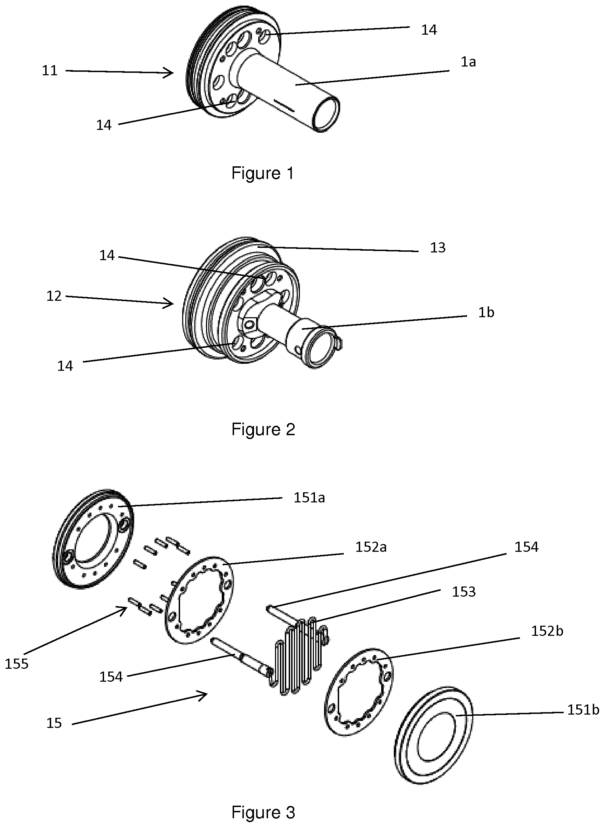

Figures 1 and 2 show the chamber 1 according to the invention, the chamber comprising:
- an upper part 11 and a lower part 12 with a ring 13 for closing and holes 14 for pins through which the heaters 15a, 15b receive the voltage needed for heating, wherein all holes 14 are provided with airtight seals, which withstand heating up to 300 °C;
- a filter 16 located between the upper 11 and lower part 12;
- an inlet 1a for leading the sampled air to the filter and a system of valves, which regulates the sampled air flow and air flow during the analysis;
- two heaters 15a, 15b comprising at least a housing and a heating wire, wherein the first heater is installed in the upper part and the second heater is installed in the lower part, wherein the distance of the heaters from the filter is from 1 to 10 mm, and wherein the heaters are controlled with electronics comprising at least voltage driver, pins and voltage clamps;
- preferably a temperature sensor installed in the lower part 12; and
- an outlet 1b for leading the created CO₂ out of the chamber 1 towards a CO₂ detector.

Figure 3 shows construction of each heater 15, wherein each heater 15 comprises:
- a housing comprising a first heater cover 151a and a second heater cover 151b;
- a heating wire 153 in the shape of a winding line installed between the first 152a and second heater cover 152b, wherein one heater isolator is placed between the cover and the heating wire on each side;
- two contact pins 154 welded to the heating wire 153; and
- a multitude of ceramic pins 155 mounted through the isolator 152 and installed in both heater covers 151.

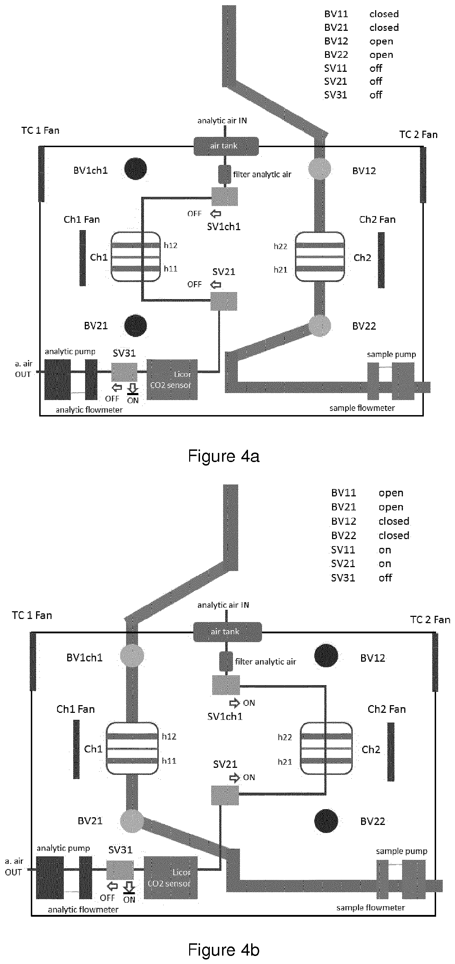

Figure 4 is a schematic view of operation of the device for measuring carbonaceous aerosol with the chamber according to the invention, wherein the first chamber is performing analysis and the second chamber is sampling (figure 4a) and vice versa (figure 4b). Figure 4a shows the flow diagram of the device, controlled by a system of valves which alternate the two channels to the common elements of pump, CO₂ analyzer, etc. The device collects the sample of atmospheric aerosols on a central spot area of a 47-mm diameter quartz fiber filter enclosed in the chamber according to the invention, at a preferred controlled sampling flow rate of 16.7 LPM, i.e. 1 m³ per hour, provided by a closed-loop-stabilized internal pump. The second chamber Ch2 is performing sampling, therefore the first ball valve BV12 is open and also the second ball valve BV22 is open. The first chamber Ch1 is performing analysis, wherein the ball valves BV11 and BV21 are closed and solenoid valves SV11, SV21 and SV31 are off. The analytic air is let into the first chamber Ch1, where the first heater H11 is turned on first, followed by the second heater H12. Heating is controlled with the electronics as described above, while the CH1 Fan provides cooling after the analysis. When the combustion of carbonaceous aerosols collected on the filter between both heaters H11 and H12 is concluded, the resulting CO₂ is led to the CO₂ sensor downstream of the first chamber Ch1. Figure 4b is essentially a mirror image of Figure 4a.

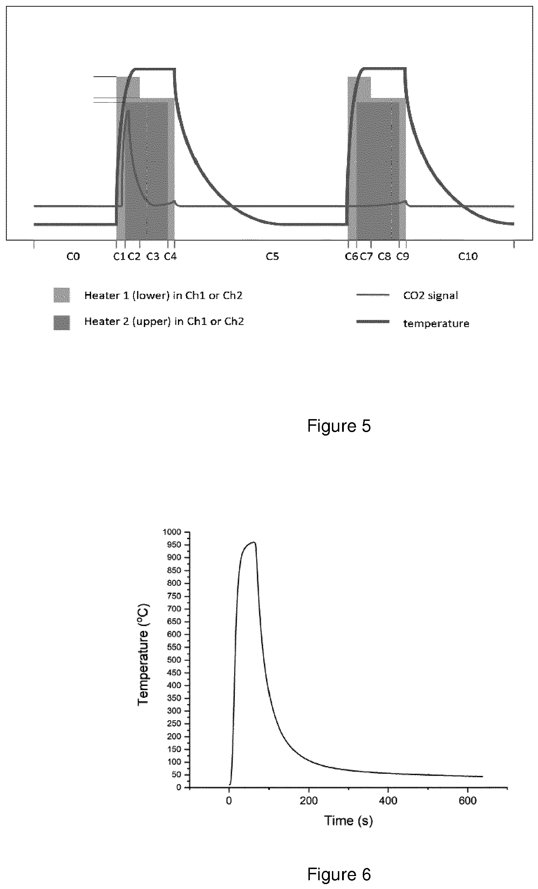

Figure 5 shows the heating of the heaters during analysis and the level of temperature and released CO₂. This figure is based on the preferred embodiment of the method of operation of the device with the chamber according to the invention, wherein heating is performed as follows:
i. heating of the first heater below the filter with a high voltage to achieve fast heating (C1);
ii. heating of the second heater above the filter (C2);
iii. adjusting the voltage on the first heater to achieve a temperature around 940 °C, which prevents overheating and unwanted degradation of the heating wire (end of C2 and beginning of C3);
iv. turning of the second heater above the filter (end of C3);
v. turning of the first heater below the filter (C4);
vi. cooling both heaters to a temperature below 50 °C (C5); and
repeating the above sequence of steps i) to vi) (C6 to C10).
In the sequence of steps the CO₂ signal increases in the first heating cycle, wherein CO₂ signal could also be present in the second cycle. C5 and C10 cycle stages are longer as the heaters take longer to cool than to heat to the required temperature.

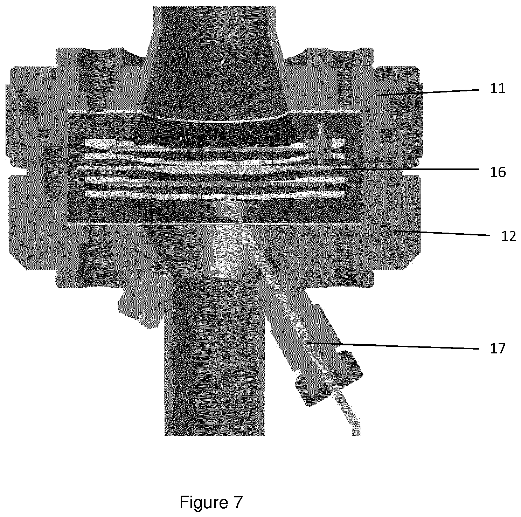

The filter reaches its final temperature around 940 °C in approximately 10 seconds, wherein the temperature rise is shown in Figure 6. The chamber is preferably equipped with a temperature sensor 16 for measuring temperature below the filter, wherein the said sensor 17 is usually placed in the lower part 12 of the chamber as shown in Figure 7.

The newly developed chamber and the device for measuring carbonaceous aerosols according to the invention enables measurement of the concentrations of total aerosol carbon continuously with high time resolution as rapid as 20 min. Two parallel flow channels provided with the improved chamber allow continuous operation: while one channel analyzes, the other collects the next sample. Thermal analysis by flash-heating of the sample collected on a quartz fiber filter inside the chamber efficiently converts all the particulate carbon to CO₂. The increase in CO₂ concentration above baseline in a flow of analytic air is measured by an integrated NDIR detector. When the device according to the invention is combined with an AE33 Aethalometer or a similar device for measuring black carbon, the TC-BC method yields OC-EC data with much greater time resolution than that offered by the analysis of filter-based samples.

## Claims

1. A heating chamber for a device for measuring carbonaceous aerosol, **characterized in that** the chamber comprises:
- housing with two heaters encasing a filter, the heaters being controlled with suitable electronics,
wherein
∘ the housing and the heaters are made of stainless steel,
∘ one of the said heaters is placed above the filter, while the other heater is placed below the filter, and
∘ the distance of the heaters from the filter is from 1 to 10 mm?

2. The heating chamber according to claim 1, **characterized in that** it comprises at least the following:
- an upper part and a lower part with a ring for closing and holes for pins through which the heaters receive the voltage needed for heating, wherein all holes are provided with airtight seals, which withstand heating up to 300 C;
- a filter located between the upper and lower part;
- an inlet for leading the sampled air to the filter and a system of valves, which regulates the sampled air flow and air flow during the analysis;
- two heaters comprising at least a housing, pins for connection with electronics controlling heating welded to a heating wire, wherein the first heater is installed in the upper part and the second heater is installed in the lower part, wherein the distance of the heaters from the filter is from X to W mm, and wherein the heaters are controlled with electronics comprising at least a voltage driver and voltage clamps; and
- an outlet for leading the created CO₂ out of the chamber towards a CO₂ detector.

3. The heating chamber according to claim 1 or claim 2, **characterized in that** each heater comprises a steel housing also serving as the holder of the filter, wherein the following components are housed within the said housing:
- the heating wire shaped as winding line to cover as much surface of the filter as possible;
- suitably shaped ceramics with small pins for protecting the heating wire from short circuits due to the metal (steel) housing; and
- at least one pin welded to the heating wire for connection to the electronics in order to allow heating of the heating pin by applying suitable voltage.

4. The heating chamber according to any of the preceding claims, **characterized in that** the heating wire is made of material that does not release carbon upon heating, the preferred option being ferritic iron-chromium-aluminium alloy.

5. The heating chamber according to any of the preceding claims, **characterized in that** said heaters are controlled by electronics, the main component of the latter being a voltage driver, one for each of the heaters, wherein the voltage driver through the contact and ceramic pins of the heaters regulates the voltage on the heating wire with 0.1 V precision, wherein the power of the heaters is measured with a voltage clamp.

6. The heating chamber according to the preceding claim, **characterized in that** said electronics turn on the lower heater before the upper heater.

7. The heating chamber according to any of the preceding claims, **characterized in that** said electronics are arranged to control the heaters as follows:
i. heating of the first heater below the filter with a high voltage to achieve fast heating;
ii. heating of the second heater above the filter;
iii. adjusting the voltage on the first heater to achieve a temperature around 940 °C, which prevents overheating and unwanted degradation of the heating wire;
iv. turning of the second heater above the filter;
v. turning of the first heater below the filter; and
vi. cooling both heaters to a temperature below 50 °C,
wherein step iii) may also be performed immediately after step i) or at the same time as step ii).

8. The heating chamber according to the preceding claim, **characterized in that** the electronics repeat the sequence of steps i) to vi).

9. The heating chamber according to any of the preceding claims, **characterized in that** said filter is any filter stable at temperatures up to 1100 °C, preferably a quartz fibre filter.

10. The heating chamber according to any of the preceding claims, **characterized in that** the chamber is equipped with a temperature sensor for measuring temperature below the filter, wherein the said sensor is usually placed in the lower part of the chamber.

11. A device for quantification of carbonaceous aerosols comprising at least one, preferably two parallel flow channels, each provided with one chamber according to any of the preceding claims, wherein one channel is collecting samples and the other channel is analysing the sample collected during a previous sampling period; and further comprising a CO₂ detector located downstream of the chambers to measure the amount of CO₂ formed during combustion of sampled air.

12. The method of operation of the device according to the preceding claim, the method comprising the following steps:
a) collecting a sample of atmospheric aerosols on the quartz fibre filter enclosed in the stainless-steel chamber, preferably at a controlled sampling flow rate of 16.7 LPM, wherein the sampling time is from 15 minutes to 24 hours, preferably 60 minutes,
b) combusting the sample from step a) with two flash-heating elements to convert all carbonaceous compounds into CO₂, wherein the first heater below the filter is turned on first and the second heater above the filter is turned on after the first heater, followed by a preferred step of adjusting the voltage on the first heater;
c) detecting in step b) created CO₂ by the NDIR CO₂ detector, wherein the background level of CO₂ in ambient air during the heating cycle is determined before and after the heating cycle to provide the baselines against which the combustion pulse is measured, and
d) cooling the chamber and combustion elements after analysis, wherein cooling is enabled with at least one fan located outside of the chamber in the device, where the chamber is installed.

13. The method according to the preceding claim, **characterized in that** step b) is performed as follows:
i. heating of the first heater below the filter with a high voltage to achieve fast heating;
ii. heating of the second heater above the filter;
iii. adjusting the voltage on the first heater to achieve a temperature around 940 °C, which prevents overheating and unwanted degradation of the heating wire;
iv. turning of the second heater above the filter;
v. turning of the first heater below the filter; and
vi. cooling both heaters to a temperature below 50 °C;
vii. wherein step iii) may also be performed immediately after step i) or at the same time as step ii).

14. The method according to the preceding claim, **characterized in that** after step vi) the sequence of steps i) to vi) is repeated one more time.

15. Use of the heating chamber according to any of the claims from 1 do 10, the device according to claim 11 and/or the method according to any of the claims from 12 to 14 in environmental monitoring, especially in measuring carbonaceous aerosols.
